# FASCICULE DE BREVET EUROPEEN

(11) **EP 0 947 752 B1**
(45) Date de publication et mention de la délivrance du brevet: **31.07.2002**
(21) Numéro de dépôt: 99410018.8
(22) Date de dépôt: 19.03.1999
(51) Int. Cl.: F16K 15/14, A61M 27/00

(54) **Valve à seuil**
Schwellwertventil
Threshold valve

(30) Priorité: 30.03.1998 FR 9804210
(43) Date de publication de la demande: 06.10.1999
(73) Titulaire: Medex, 74940 Annecy le Vieux (FR)
(72) Inventeur: Lacroix, Jean-Pierre, 74940 Annecy le Vieux (FR)
(74) Mandataire: Gasquet, Denis

(56) Documents cités:
- EP-A- 0 334 157
- DE-U- 29 519 322
- DE-U- 29 709 021
- US-A- 4 535 820
- US-A- 4 781 674
- US-A- 5 277 171

## Description

La présente invention concerne pour une valve du type haute pression et plus particulièrement une valve à seuil destinée à être utilisée en combinaison avec un dispositif pour l'injection médicale de liquide.

Les développements récents de la médecine ont conduit à mettre au point différents procédés d'analyse et de contrôle de l'état des patients. Parmi ces procédés il existe les analyses faites après injection d'un produit de contraste. C'est par exemple le cas pour les analyses du type artériographique qui consistent à injecter un produit iodé dans les vaisseaux pour opacifier l'ensemble de ceux-ci et ainsi, par radio, déterminer les anomalies éventuelles, comme les thromboses, les embolies, les compressions et autres....

On connaît des injecteurs destinés à injecter des produits de contraste et comprenant une tête d'injection -reliée à une ou plusieurs aiguilles par un conduit tubulaire permettant le passage du liquide de contraste de la seringue proprement dite à l'aiguille d'injection. On injecte par exemple dans les vaisseaux sanguins d'un patient des produits iodés. Les injecteurs sont utilisés généralement pour plusieurs patients, et quand on connaît les risques de contamination, il est d'importance fondamentale d'interdire au sang du patient de remonter dans les conduits et contaminer les éléments de l'injecteur non jetable. La solution actuelle au problème consiste à placer une valve antiretour à seuil sur le conduit d'injection.

Dans le cas d'une utilisation de valve pour une injection veineuse, la pression du liquide est d'environ 10 bars, aussi la valve telle que décrite dans le brevet américain US 4.535 820 ou similaire et comprenant un corps de valve réalisé en plusieurs éléments liés entre eux par soudage ou collage semble convenir. Toutefois ce type de valve présente un inconvénient majeur, dans le cas d'injection à haute pression tel qu'en injection artérielle comme par exemple en angiographie traditionnelle ou numérisée. En effet en cas de forte pression, des fuites peuvent se produire par exemple aux zones de liaison des différents éléments constituant le corps de valve.

On connaît également d'autres valves, telle que celle décrite dans le document US 5 277 171, ladite valve présentant un corps de renfort tubulaire. Toutefois, ce type de valve ne peut supporter les hautes pressions utilisées en injection artérielle.

La présente invention a pour objet de solutionner les inconvénients précédemment évoqués et propose une construction de valve à seuil antiretour, particulièrement simple et économique, qui résiste à des hautes pression.

Ainsi, la valve à seuil antiretour selon l'invention comprend un corps de renfort enveloppant au moins en partie le corps de valve, ledit corps de valve étant constitué au moins par un corps amont et un corps aval liés ensemble, le corps de renfort comprenant une paroi périphérique centrale enveloppant au moins la zone d'assemblage des éléments constituant le corps de valve, et est caractérisée en ce que le corps de renfort comprend par ailleurs une paroi périphérique amont enveloppant une portion tubulaire amont du corps amont et une paroi périphérique aval enveloppant une portion tubulaire aval du corps aval ainsi qu'une paroi amont transversale de retenue et une paroi aval transversale de retenue, pour bloquer les différents éléments du corps de valve dans le sens longitudinal.

Selon une caractéristique complémentaire le corps de valve comprend au moins un corps central disposé entre le corps amont et le corps aval.

Ajoutons que le corps de valve ainsi que le corps de renfort sont réalisés en matière plastique.

L'invention concerne aussi le procédé de réalisation, qui est caractérisé en ce que le corps de renfort est surmoulé autour du corps de valve.

Le corps de renfort de l'invention peut bien entendu être surmoulé sur tout type de valve, ce qui permet de transformer une valve classique en une valve pouvant subir de fortes pressions. Notons aussi que le corps de renfort permet aussi de ne pas avoir à solidariser les différents éléments du corps de valves, leur solidarisation se faisant alors lors du surmoulage du corps de renfort.

D'autres caractéristiques et avantages de l'invention se dégageront de la description qui va suivre en regard des dessins annexés qui ne sont donnés qu'à titre d'exemples non limitatifs.

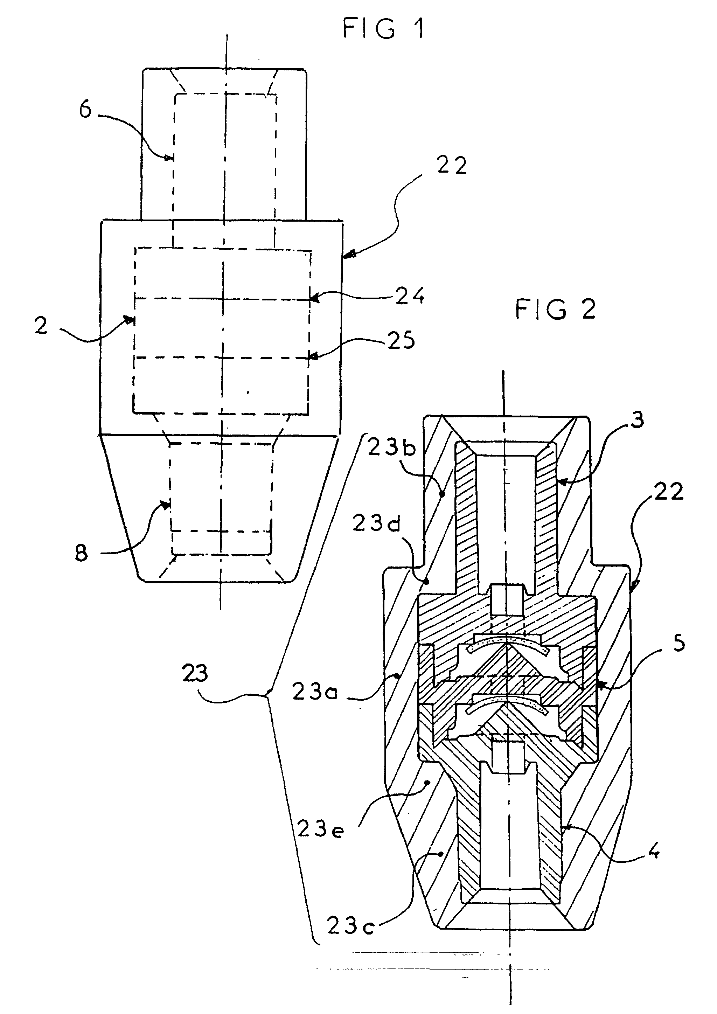

Les figures 1 à 5 illustrent un mode de réalisation d'une valve selon l'invention.

La figure 1 est une vue latérale extérieure.

La figure 2 est une vue en coupe longitudinale.

La figure 3 est une vue éclatée du corps de valve proprement dit.

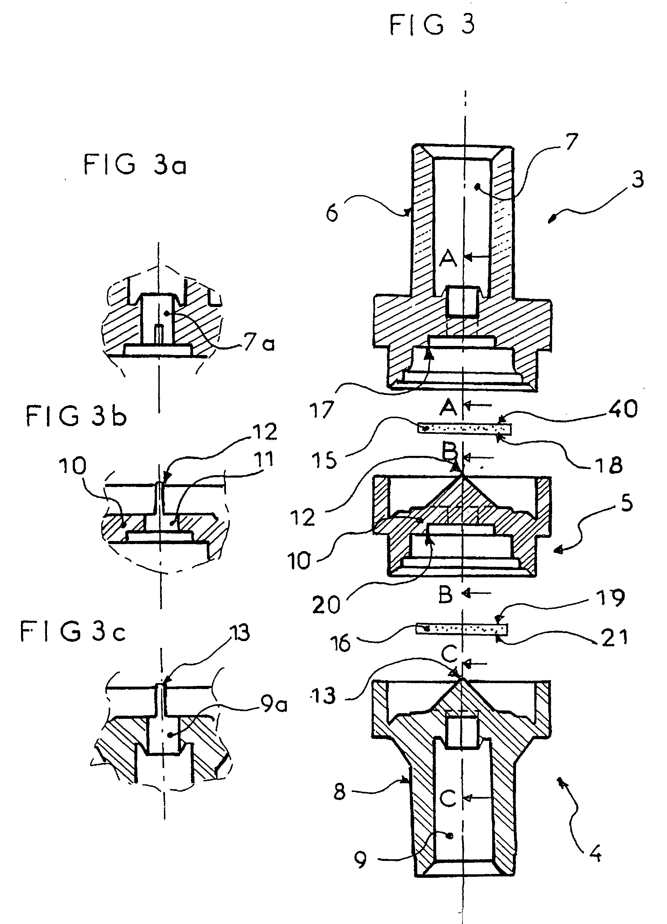

La figure 3 est une vue en coupe partielle selon A-A.

La figure 3b est une vue en coupe partielle selon B-B.

La figure 3c est une vue en coupe partielle selon C-C.

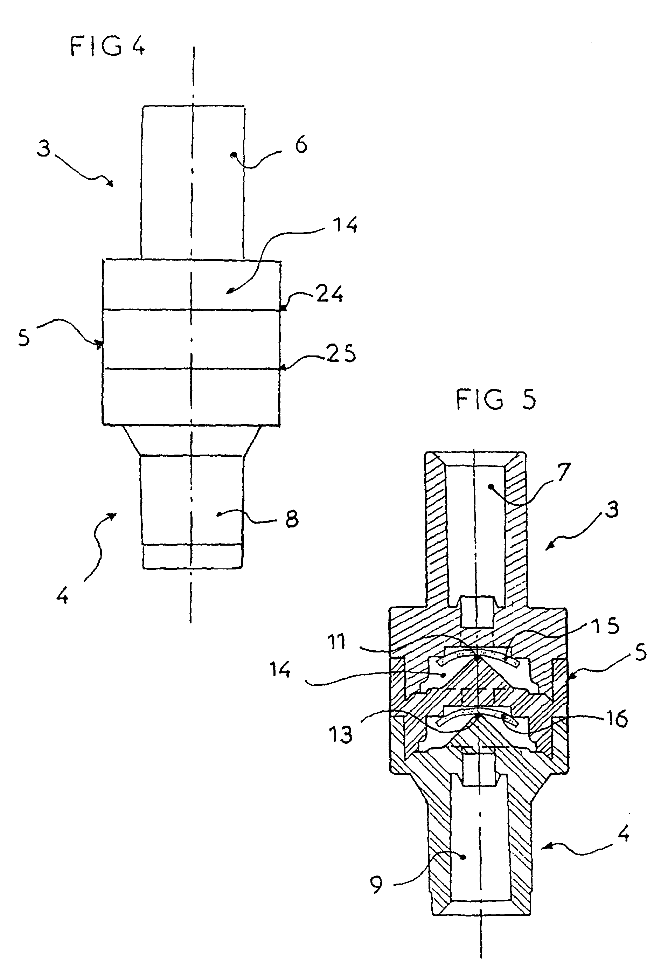

La figure 4 est une vue extérieure du corps de la valve proprement dite.

La figure 5 est une vue en coupe longitudinale de la figure 4.

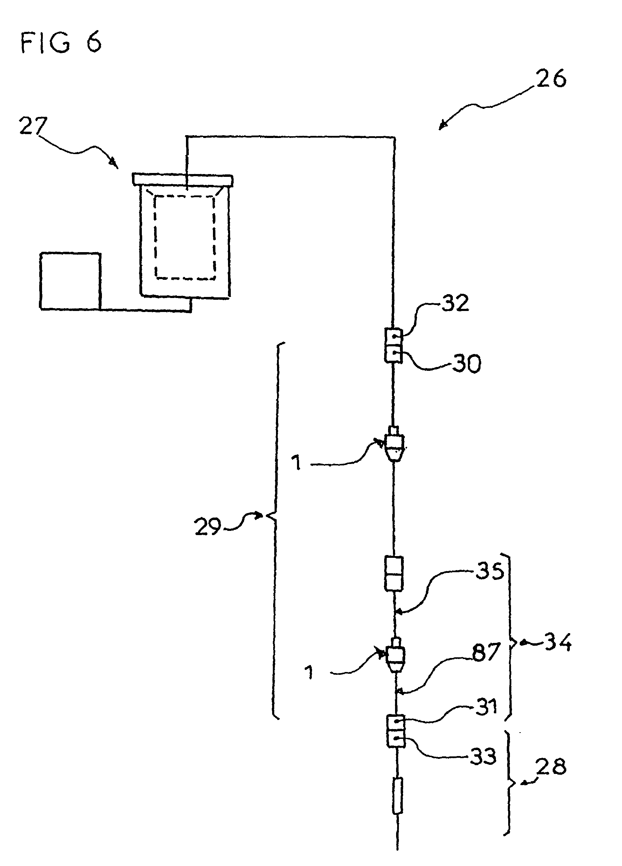

La figure 6 est une vue générale schématique d'un dispositif d'injection utilisant la valve de l'invention

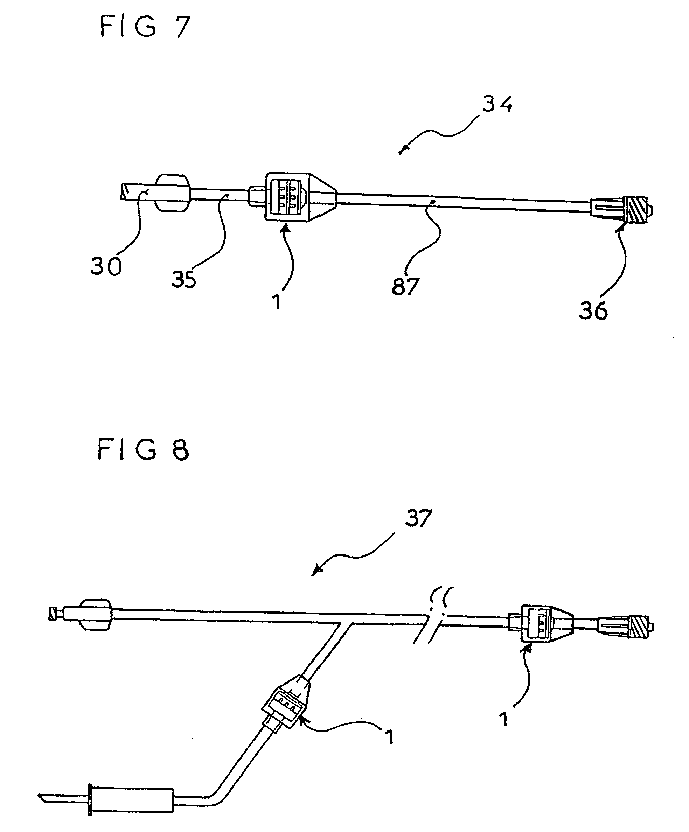

Les figures 7, 8, 9 et 9a illustrent différents kits utilisés dans des dispositifs d'injection.

La valve proprement dite portant la référence générale (1) comprend de façon connue en soi, un corps de valve (2) constitué d'un corps amont de valve (3) et d'un corps aval de valve (4), tandis qu'au moins un corps central de valve (5) est disposé entre ledit corps amont et ledit corps aval. Chacun des corps, amont et aval, comprend une portion d'extrémité tubulaire percée d'un canal central. Ainsi, le corps amont (3) comprend une portion tubulaire amont (6) percée d'un canal amont central ou entrée (7) tandis que le corps aval (4) comprend une portion tubulaire aval (8) percée d'un canal aval central (9) ou sortie.

Le corps central de valve (5) comprend une paroi transversale (10) comprenant un trou central (11) et un premier pointeau central (12) s'étendant vers le corps amont. Par ailleurs, le corps aval (4) comprend un second pointeau central (13) s'étendant vers la paroi transversale (10) du corps central. L'ensemble constitué par le corps amont (3) et le corps aval (4) forment avec le corps central, une chambre d'obturation (14) dans laquelle s'étend la paroi transversale (10) du corps central (5).

Par ailleurs la chambre d'obturation (14) contient deux disques obturateur (15, 16) un premier disque (15) et un deuxième disque (16).

Notons que le premier disque (15) ou disque amont est tel que sa face amont (40) est en appui sur un siège à arête vive (17) réalisée dans le corps amont, tandis que sa face aval (18) est sollicitée par le premier pointeau central (12).

De même, le deuxième disque (16) ou disque aval est tel que sa face amont (19) est en appui sur un siège à arête vive (20) réalisée dans la paroi transversale du corps central, tandis que sa face aval (21) est sollicitée par le second pointeau (13). Sous l'action des deux pointeaux respectifs (12, 13), les deux disques obturateurs (15, 16) sont plaqués contre leurs sièges correspondants (17, 20). On comprend donc que les disques obturateurs (15, 16) interdisent le passage du liquide depuis la sortie (9) vers l'entrée (7) car toute surpression dans la sortie (9) plaque les disque sur leurs sièges.

Par contre, la déformation élastique des disques obturateurs (15, 16) permet le passage du liquide depuis l'entrée (7) vers la sortie (9) lorsque la surpression à l'entrée (7) est suffisante pour déformer les disques et les écarter de leur siège. Ajoutons que le réglage du seuil de passage du liquide est réalisé par le choix approprié des dimensions et de la matière des disques obturateurs, ainsi que par la précontrainte donnée à ou aux obturateurs

L'assemblage des différents éléments du corps de valve (3, 4, 5) se fait par collage, ou soudage, ou toute autre technique.

Par ailleurs, le corps amont (3) le corps aval (4) et le corps central (5) sont réalisés en matière plastique conformes aux exigences du domaine médical comme par exemple en acrylobutadienstyrène communément dénommé ABS.

Selon l'invention la valve (1) comprend un corps de renfort (22) enveloppant au moins une partie du corps de valve. Le corps de renfort (22) constitue une enveloppe périphérique externe monobloc et comprend une paroi périphérique de renfort (23). Ainsi il comprend une paroi périphérique centrale (23a) enveloppant au moins la zone d'assemblage des éléments constituant le corps de valve et en particulier, la zone d'assemblage (24) du corps- amont (3) et du corps central (5) et la zone d'assemblage (25) du corps central (5) avec le corps aval (4) Le corps de renfort (22) comprend par ailleurs une paroi périphérique amont (23b) enveloppant la portion tubulaire amont (6) du corps amont (3) et une paroi périphérique aval (23c) enveloppant la portion tubulaire aval (8) du corps aval (4). Ajoutons que le corps de renfort comprend une paroi amont transversale de retenue (23d) et une paroi aval transversale de retenue (23e), pour bloquer les différents éléments du corps de valve dans le sens longitudinal.

Comme nous l'avons vu précédemment, la valve proprement dite (1) est constituée de plusieurs éléments liés ensemble, et par exemple trois éléments comme selon le mode d'exécution donné à titre d'exemple, à savoir un corps amont (3), un corps aval (4) et un corps central (5). Chacun des éléments sont réalisés séparément par exemple par injection de matière plastique tel qu'en A.B.S. ou tout autre matière appropriée pour être assemblés l'un à l'autre par collage ou soudage. Le corps de renfort (22), selon une caractéristique de l'invention est surmoulé sur la valve proprement dite, le surmoulage se faisant avec une matière plastique appropriée et compatible avec la matière avec laquelle est réalisée la valve proprement dite. Ainsi, le corps de renfort (22) peut être réalisé en A.B.S.

La valve (1) selon l'invention résiste grâce à son corps de renfort (22) à des pressions importantes comme par exemple à des pressions de liquide et d'injection de 80 bars et peut être utilisée pour tout type d'injecteur de liquide médical et plus particulièrement à ceux utilisés en angiographie ou en coronographie.

La valve (1) selon l'invention peut être utilisée par exemple, dans un dispositif d'injection (26) de liquide médical tel qu'illustré à la figure 6. Ce dispositif pouvant être par exemple celui décrit dans la Demande de Brevet n° 9700235 déposée par la demanderesse et qui comprend un injecteur proprement dit où le dispositif d'alimentation (27) est relié à un conduit d'injection (28) tel qu'un cathéter, une aiguille hypodermique ou intraveineuse par l'intermédiaire d'une canalisation (29) constituée de plusieurs éléments. Ladite canalisation (29) comprend, à ses extrémités, deux raccords de branchement, un raccord amont (30) et un raccord aval (31). le raccord amont (30) est destiné à être raccordé à un raccord d 'alimentation (32) solidaire de l'injecteur, tandis que le raccord aval (31) est destiné à être raccordé au raccord amont (33) du conduit d'injection (28). Par ailleurs et avantageusement, la canalisation (29) comprend un dispositif de sécurité (34).

La valve selon l'invention, peut être utilisée dans un raccord amovible (34) constituant le dispositif de sécurité du type de celui décrit dans la demande de brevet française numéro 93 12590 publiée sous le numéro 2 711 319 et illustré plus particulièrement à la figure 7, et qui comprend une canalisation amont (35) munie d'une connexion d'entrée (30) pour son raccordement à la sortie d'un dispositif d'alimentation en liquide et une canalisation aval (87) munie d'une connexion de sortie (36) pour son raccordement à l'entrée d'un conduit d'injection.

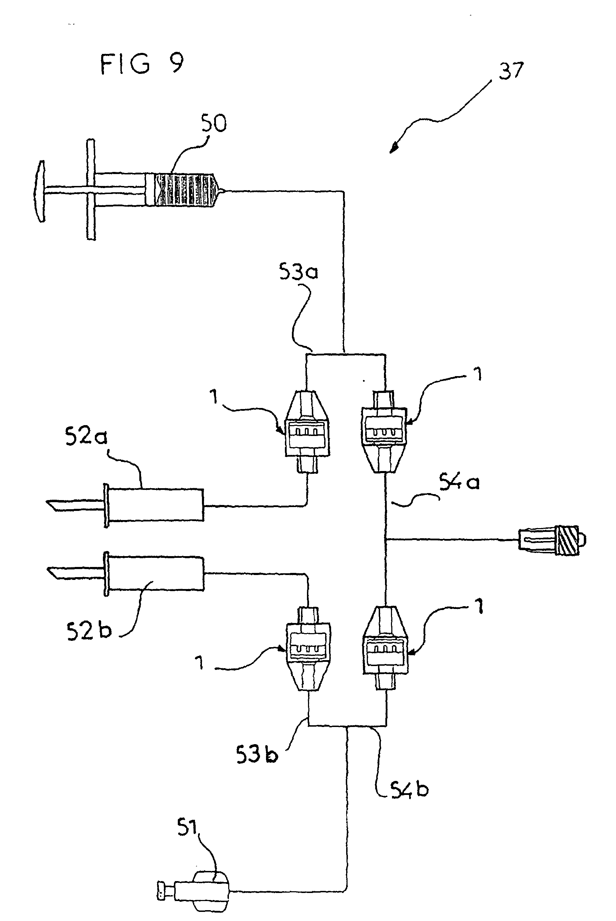

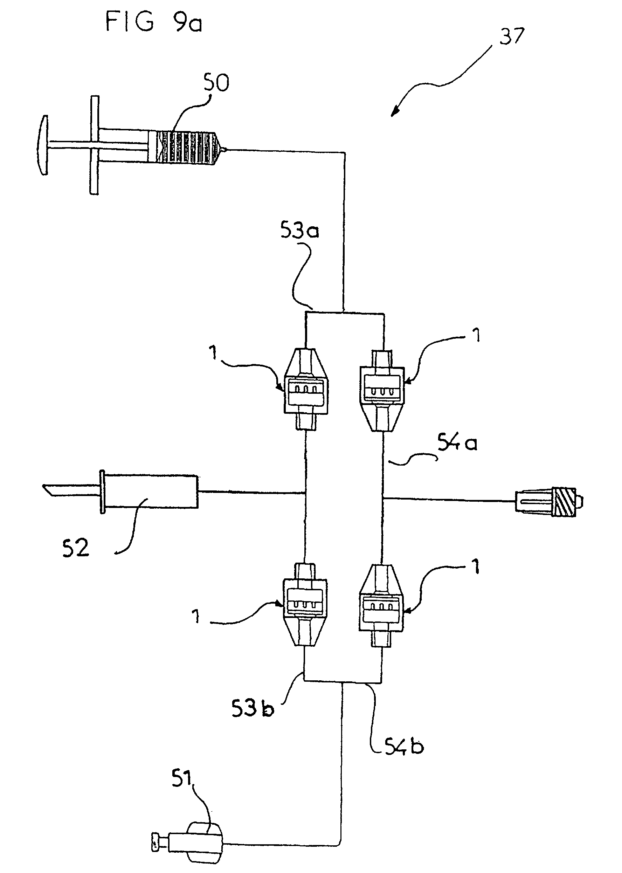

On peut utiliser les valves haute pression renforcées (1) de l'invention dans des kits de remplissage (37) destinés à l'angiographie tel qu'illustré à la figure 8 ou à la figure 9. A la figure 9, est illustré un kit destiné a être utilisé pour les examens coronographiques, et qui comprend, d'un côté, une seringue manuelle (50) et, de l'autre côté, une connexion (51) destinée à relier le kit (37) à une seringue automatique non représentée; Ledit kit (37) comprend par ailleurs un ou plusieurs flacons de remplissage (52a, 52b), tandis que chacune des canalisations de remplissage (53a, 53b) comprend une valve (1) selon l'invention. De même chacune des canalisations d'injection (54a, 54b) comprend une valve (1) de l'invention.

La figure 9a est une variante d'exécution de la figure 9, selon laquelle le kit ne comprend qu'un seul flacon de remplissage (52).

La valve donnée à titre d'exemple comprend deux disques obturateurs (15, 16), cela permet d'avoir une meilleure sécurité pour l'effet antiretour, mais on peut utiliser avec le corps de renfort, une valve comprenant un seul disque obturateur, ou plus de deux disques obturateurs.

On a compris par la description faite de l'invention que la valve (1) avec son corps de valve (2, 3, 4, 5) constitue une valve classique avec sa portion d'extrémité tubulaire amont (6) et aval (8), susceptible de fonctionner même en l'absence du corps de renfort (22) de l'invention on notera que le corps de renfort enveloppe non seulement le corps de valve proprement dit mais aussi les portions d'extrémité tubulaires amont et aval

## Revendications

1. Valve à seuil antiretour (1) comprenant un corps de renfort (22) enveloppant au moins en partie le corps de valve (2), ledit corps de valve étant constitué au moins par un corps amont (3) et un corps aval (4) liés ensemble, le corps de renfort (22) comprenant une paroi périphérique centrale (23a) enveloppant au moins la zone d'assemblage (24, 25) des éléments constituant le corps de valve (2), **caractérisée en ce que** le corps de renfort (22) comprend par ailleurs une paroi périphérique amont (23b) enveloppant une portion tubulaire amont (6) du corps amont (3) et une paroi périphérique aval (23c) enveloppant une portion tubulaire aval (8) du corps aval (4) ainsi qu'une paroi amont transversale de retenue (23d) et une paroi aval transversale de retenue (23e), pour bloquer les différents éléments du corps de valve dans le sens longitudinal.

2. Valve à seuil antiretour (1) selon la revendication 1 **caractérisée en ce que** le corps de renfort (22) constitue une enveloppe périphérique externe monobloc.

3. Valve à seuil antiretour (1) selon l'une quelconque des revendications précédentes **caractérisée en ce que** le corps de valve (2) est constitué d'au moins trois éléments (3, 4, 5) liés ensemble.

4. Valve à seuil antiretour (1) selon la revendication 3 **caractérisée en ce que** le corps de valve (2) comprend au moins un corps central (5) disposé entre le corps amont (3) et le corps aval (4) et relié avec ceux-ci.

5. Valve à seuil antiretour (1) selon une quelconque des revendications précédente **caractérisée en ce que** le corps de valve (2) ainsi que le corps de renfort (22) sont réalisés en matière plastique.

6. Valve à seuil antiretour (1) selon l'une quelconque des revendications précédentes **caractérisée en ce que** le corps de renfort (22) est surmoulé autour du corps de valve (2).

7. Kit de remplissage (37) **caractérisé en ce qu'**il comporte une valve à seuil antiretour (1) selon l'une quelconque des revendications précédentes disposée sur les canalisations (53a, 53b, 54a, 54b) du kit, ledit kit comprenant d'un côté une seringue manuelle (50) et de l'autre côté une connexion (51) destinée à le relier à une seringue automatique, ainsi qu'un ou plusieurs flacons de remplissage (52a, 52b).

## Patentansprüche

1. Schwellwert-Rückschlagventil (1) umfassend einen Verstärkungskörper (22), der zumindest teilweise den Ventilkörper (2) umgibt, wobei der Ventilkörper mindestens aus einem oberen Körper (3) und aus einem unteren Körper (4) besteht, welche untereinander verbunden sind, wobei der Verstärkungskörper (22) eine umlaufende zentrale Wandung (23a) aufweist, die mindestens den Bereich (24, 25) umgibt, in dem die Elemente, aus denen der Ventilkörper (2) besteht, zusammengefügt werden, **dadurch gekennzeichnet, dass** der Verstärkungskörper (22) außerdem eine obere, einen oberen rohrförmigen Abschnitt (6) des oberen Körpers (3) umgebende Wandung (23b) und eine untere, einen unteren rohrförmigen Abschnitt (8) des unteren Körpers (4) umgebende Wandung (23c), sowie eine obere, quer angeordnete Haltewand (23d) und eine untere quer angeordnete Haltewand (23e) zum Blockieren der verschiedenen Elemente des Ventilkörpers in Längsrichtung umfasst.

2. Schwellwert-Rückschlagventil (1) nach Anspruch 1, **dadurch gekennzeichnet, dass** der Verstärkungskörper (22) als einstückiges umlaufendes äußeres Gehäuse ausgebildet ist.

3. Schwellwert-Rückschlagventil (1) nach einem der vorstehenden Ansprüche, **dadurch gekennzeichnet, dass** der Ventilkörper (2) aus mindestens drei miteinander verbundenen Elementen (3, 4, 5) besteht.

4. Schwellwert-Rückschlagventil (1) nach Anspruch 3, **dadurch gekennzeichnet, dass** der Ventilkörper (2) mindestens einen zentralen Körper (5) umfasst, welcher zwischen dem oberen Körper (3) und dem unteren Körper (4) angeordnet ist und mit diesen verbunden ist.

5. Schwellwert-Rückschlagventil (1) nach einem der vorstehenden Ansprüche, **dadurch gekennzeichnet, dass** sowohl der Ventilkörper (2) als auch der Verstärkungskörper (22) aus Kunststoff sind.

6. Schwellwert-Rückschlagventil (1) nach einem der vorstehenden Ansprüche, **dadurch gekennzeichnet, dass** der Verstärkungskörper (22) um den Ventilkörper (2) ausgeformt ist.

7. Einfülleinheit (37), **dadurch gekennzeichnet, dass** sie ein an den Schläuchen (53a, 53b, 54a, 54b) der Einheit angebrachtes Schwellwert-Rückschlagventil (1) nach einem der vorstehenden Ansprüche aufweist, wobei die Einfülleinheit auf der einen Seite eine manuelle Spritze (50) und auf der anderen Seite ein Verbindungsstück (51) zum Anschließen an die automatische Spritze sowie eine oder mehrere Füllflaschen (52a, 52b) umfasst.

## Claims

1. Valve with a nonreturn threshold (1) comprising a reinforcing body (22) at least partly enveloping the valve body (2), said valve body being made up of at least an upstream body (3) and a downstream body (4) joined together, the reinforcing body (22) comprising a central peripheral wall (23a) enveloping at least the assembly zone (24, 25) of the elements constituting the valve body (2), **characterised in that** the reinforcing body (22) further comprises an upstream peripheral wall (23b) enveloping an upstream tubular portion (6) of the upstream body (3) and a downstream peripheral wall (23c) enveloping a downstream tubular portion (8) of the downstream body (4) as well as an upstream transverse retaining wall (23d) and a downstream transverse retaining wall (23e), to block the different elements of the valve body in the longitudinal direction.

2. Valve with a nonreturn threshold (1) according to claim 1, **characterised in that** the reinforcing body (22) constitutes a one-piece outer peripheral casing.

3. Valve with a nonreturn threshold (1) according to one of the preceding claims, **characterised in that** the valve body (2) consists of at least three elements (3, 4, 5) joined together.

4. Valve with a nonreturn threshold (1) according to claim 3, **characterised in that** the valve body (2) comprises at least one central body (5) disposed between the upstream body (3) and the downstream body (4) and connected thereto.

5. Valve with a nonreturn threshold (1) according to any one of the preceding claims, **characterised in that** the valve body (2) and the reinforcing body (22) are made of plastics.

6. Valve with a nonreturn threshold (1) according to one of the preceding claims, **characterised in that** the reinforcing body (22) is overmoulded around the valve body (2).

7. Refill kit (37), **characterised in that** it comprises a valve with a nonreturn threshold (1) according to any one of the preceding claims, fitted on tubing (53a, 53b, 54a, 54b) in the kit, eaid kit comprising on the one hand a manual syringe (50) and on the other hand a connection (51) intended to connect it to an automatic syringe, as well as one or more refill bottles (52a, 52b).
